# EUROPEAN PATENT APPLICATION

(11) **EP 3 085 360 A1**
(43) Date of publication of application: **26.10.2016**
(21) Application number: 15305592.6
(22) Date of filing: 20.04.2015
(51) Int. Cl.: A61K 9/127, A61K 49/00, A61K 9/51, A61K 31/355, A61K 31/5585

(54) **LIPID BASED NANOCARRIER COMPOSITIONS LOADED WITH METAL NANOPARTICLES AND THERAPEUTIC AGENT**

(71) Applicant: UNIVERSITE DE BORDEAUX, 33000 Bordeaux (FR); INSTITUT NATIONAL DE LA SANTE ET DE LA RECHERCHE MEDICALE (INSERM), 75654 Paris Cédex 13 (FR); CENTRE NATIONAL DE LA RECHERCHE SCIENTIFIQUE -CNRS-, 75016 Paris (FR)
(72) Inventor: Barthelemy, Philippe, 33700 MERIGNAC (FR); Oumzil, Khalid, 33700 MERIGNAC (FR); Clofent-Sanchez, Gisèle, 33290 LE PIAN MEDOC (FR); Jacobin-Valat, Marie-Josée, 33650 SAINT-MORILLON (FR); Laroche-Traineau, Jeanny, 33160 SAINT AUBIN DE MEDOC (FR); Mornet, Stéphane, 33440 SAINT VINCENT DE PAUL (FR); Gaudin, Karen, 33800 BORDEAUX (FR)
(74) Representative: Santarelli

(57) **Abstract**

The invention relates to non-polymeric lipid-based nanocarrier compositions loaded with metal nanoparticles and at least one therapeutic agent, useful .as agents for transportation, vectorization, cellular delivery cellular targeting or cellular localization of at least one therapeutic agent.

## Description

The invention relates to non-polymeric lipid-based nanocarrier compositions loaded with metal nanoparticles and at least one therapeutic agent.

The nanoparticle technology has allowed the implementation of novel multifunctional nanoparticles for disease, detection, therapy and treatment monitoring.

Their numerous advantages include minimizing the amount of drug needed, increasing the bioavailability, in particular for hydrophobic drugs, and reducing the drug toxicity.

Magnetic Resonance Imaging (MRI) is a worldwide-used non-invasive imaging and diagnostic technique, which is very efficient for imaging soft tissues and provides detailed anatomical images of the body. Ultrasmall superparamagnetic iron oxide nanoparticles are currently used as contrast agent in magnetic resonance imaging.

The theranostic approach refers to molecular/macromolecular targeting vectors and nano-platform technologies that incorporate both diagnostic and therapeutic functionalities. These entities can be used for simultaneous targeted drug delivery and release, as well as diagnosis, which includes monitoring disease progression and response to therapy.

In addition, this approach can be used for selecting a safe and efficient dose of therapeutic agent, recognizing adverse effects at an early stage of therapy and real-time monitoring of therapy, making it possible to adapt the treatment to the individual patients as a function of the inter-individual variability in their therapeutic responses.

Quantum dot lipid oligonucleotide bioconjugates are studied in A. Aimé et al., Bioconjugate Chem., 2013, 24, 1345-1355. These bioconjugates consist of fluorescent semiconductor nanocrystals, named quantum dots, encapsulated by nucleolipids and lipid oligonucleotide conjugates, with a view to providing functionalized quantum dots with recognition and detection properties.

It has now been found that lipid-based theranostic systems loaded with nanoparticles and a therapeutic agent, which are non-polymeric systems, could be used, in particular, for drug targeting, drug delivery and treatment monitoring, while avoiding the side-effects usually related to the use of toxic active ingredients, in particular in cancer therapy.

Advantageously, the use of nucleolipids in these systems allows the formation of stable supramolecular constructs.

Without wishing to be bound by theory, it can be hypothesized that the metal nanoparticles are associated to each other as clusters by hydrophilic/hydrophobic interactions with the nucleolipids. Said nucleolipids also serve to entrap the therapeutic agent(s), through hydrophilic/hydrophobic interactions and are present at the interface with the outer aqueous medium.

Advantageously, it has also been found that the lipid-based nanocarrier composition according to the invention allows a high content in active principle and the formation of nanoparticles which can be used for imaging purpose.

The invention thus relates to a lipid-based nanocarrier composition comprising
a) at least one compound of formula (I) 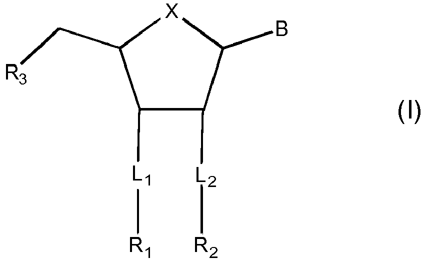 in which
   - X is an oxygen atom, a sulfur atom or a methylene group,
   - B is a purine or pyrimidine base, or else a non-natural mono- or bi-cyclic heterocyclic base, each ring of which comprises 4 to 7 members, optionally substituted ;
   - L₁ and L₂, identical or different, represent hydrogen, an oxycarbonyl -O-C(O)-group, a thiocarbamate -O-C(S)-NH- group, a carbonate -O-C(O)-O- group, a carbamate -O-C(O)-NH- group, an oxygen atom, a phosphate group, a phosphonate group or a heteroaryl group comprising 1 to 4 nitrogen atoms, unsubstituted or substituted by a linear or branched, saturated or unsaturated C₂-C₃₀ hydrocarbon chain,
   - or also, L₁ and L₂, together, form a ketal group of formula 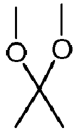
   - or also L₁ or L₂ represents hydrogen, and the other represents a hydroxy group or a heteroaryl group comprising 1 to 4 nitrogen atoms, unsubstituted or substituted by a linear or branched C₂-C₃₀ alkyl chain,
   - R₁ and R₂, identical or different, represent
   - a linear or branched C₂-C₃₀ hydrocarbon chain, saturated or partially unsaturated, or
   - a C₂-C₃₀ acyl chain,
   - a diacyl chain in which each acyl chain is C₂-C₃₀,
   - a diacylglycerol in which each acyl chain is C₂-C₃₀, or
   - a sphingosine or ceramide group in which each acyl chain is C₂-C₃₀, or
   - when L₁ or L₂ represents hydrogen, and the other represents a hydroxy group or a heteroaryl group comprising 1 to 4 nitrogen atoms, R₁ and R₂ do not exist;
   - R₃ represents
   - a hydroxy, amino, phosphate, phosphonate, phosphatidylcholine, O-alkyl phosphatidylcholine, thiophosphate, phosphonium, NH₂-R₄, NHR₄R₅ or NR₄R₅R₆ group in which R₄, R₅ and R₆, identical or different, represent a hydrogen atom or a linear or branched C₁-C₆ alkyl chain or linear or branched C₁-C₆ hydroxyalkyl, or
   - a linear or branched C₂-C₃₀ alkyl chain, optionally substituted by a hydroxy group, or
   - a heteroaryl group containing 1 to 4 nitrogen atoms, unsubstituted or substituted by a C₂-C₃₀ alkyl, optionally substituted by a hydroxy group; or by a (CH₂)ₘ-O-(CH₂)ₚ-R₉ group in which m = 1 to 6 and p = 0 to 20 and R₉ represents hydrogen or a cyclic ketal group containing 5 to 7 carbon atoms, unsubstituted or substituted by at least one linear or branched C₂-C₃₀ alkyl, or by a sterol radical,
      or
   - a -O-C(O)-(CH₂)_{q}-C(O)-O [(CH₂)₂-O]ᵣ-H group in which q is an integer from 2 to 6 and r is an integer from 4 to 30, preferably from 10 to 20, or also
   - R₃ is bound by a covalent bond to another substituent R₃, identical or different, of another compound of formula (I), identical or different, in order to form a compound of formula (I) in the form of a dimer,
      b) at least one metal nanoparticle, and
      c) at least one therapeutic agent.

By "nanocarrier" is meant that the theranostic system of the invention has an overall size (average diameter) of approximately 20 to 300 nm.

X preferably represents an oxygen atom.

By « straight or branched C₁-C₆ alkyl » is understood, for example, a methyl, ethyl, propyl, i-propyl, n-butyl, i-butyl, tert-butyl, preferably methyl or ethyl.

Preferred C₂-C₃₀ hydrocarbon chains are C₈-C₂₆, more preferably C₁₆-C₂₀ hydrocarbon chains.

Preferred linear or branched C₂-C₃₀ alkyl chains are C₈-C₂₆, more preferably C₁₆-C₂₀ linear or branched alkyl chains.

Preferred C₂-C₃₀ acyl chains are C₈-C₂₆, more preferably C₁₆-C₂₀ acyl chains.

The purine or pyrimidine base can be, for example, selected from, adenine, guanine, cytosine, xanthine, hypoxanthine, uric acid, caffeine, theobromine, uracile, thymine, dihydrouridine, and their derivatives.

Thymine and uracile are preferred.

Also, in formula (I) above, the purine or pyrimidine base can be substituted by at least one substituent selected from, for example, a halogen, an amino group, a carboxy group, a carbonyl group, a carbonylamino group, a hydroxy, azido, cyano, thiol, a C₁-C₆ straight or branched alkyl, cycloalkyl, perfluoroalkyl, alkyloxy (for example, methoxy), oxycarbonyl, vinyl, ethynyl, propynyl, acyl group etc.

By "derivatives of a purine or pyrimidine base" is meant, for example, a non-natural mono- or bi-cyclic heterocyclic base in which each cycle has 4 to 7 members, optionally substituted as stated above for the purine or pyrimidine base.

By « non-natural heterocyclic base » is meant a universal base, such as, for example, 3-nitropyrrole, 4-nitroimidazole or 5-nitroindole, which do not exist in nature.

By « heteroaryl comprising 1 to 4 nitrogen atoms » is meant a mono-or bi-cyclic carbocyclic group, aromatic or partially unsaturated, comprising 5 to 12 atoms in total, interrupted by 1 to 4 nitrogen atoms, which can be, for example, selected from furane, pyrrole, oxazole, oxadiazole, isoxazole, pyrazole, triazole, tetrazole, imidazole, pyridine, pyrimidine, pyridazine, pyrazine, benzofurane, indole, quinoleine, isoquinoleine, chromane, naphtyridine and benzodiazine groups, triazole being preferred.

The following compounds of formula (I), in which at least one condition is fulfilled, are preferred:
- X is an oxygen atom;
- B is thymine or uracile;
- L₁ and L₂ are oxycarbonyl -O-C(O)- groups which are substituted by a linear or branched C₂-C₃₀ hydrocarbon chain, preferably C₈-C₂₆, more preferably C₁₆-C₂₀, saturated or partially unsaturated;
- L₁ is a phosphate group which is substituted by diacylglycerol in which each acyl group is C₂-C₃₀, preferably C₈-C₂₆, more preferably C₁₆-C₂₀, and L₂ is hydrogen;
- R₃ is hydroxy, a NR₄R₅R₆ group in which R₄, R₅ and R₆ represent a hydrogen atom or a -O-C(O)-(CH₂)_{q}-C(O)-O [(CH₂)₂-O]ᵣ-H group in which q is an integer from 2 to 6 and r is an integer from 4 to 30, preferably from 10 to 20.

Particularly preferred compounds of formula (I) are selected from
- N-[5'-(2',3'-dioleoyl)uridine]-N',N',N'-trimethylammonium (DOTAU) (CAS Registry Number: 868226-06-6),
- Thymidine 3'-(1,2-dipalmitoyl-sn-glycero-3-phosphate), also called diC16dT(CAS Registry Number: 1160002-70-9), and
- Poly(oxy-1,2-ethanediyl), α-hydro-ω-methoxy-, ester with uridine 5'-(hydrogen butanedioate) 2',3'-di-(9Z)-9-octadecenoate (DOU-PEG) (CAS Registry Number: 1353570-75-8).

In a preferred embodiment, the lipid-based nanocarrier composition according to the invention comprises at least 2 different compounds of formula (I).

In particular, the lipid-based nanocarrier composition can comprise at least one co-lipid in addition to the compound(s) of formula (I).

By "co-lipid", is meant a compound used in combination with the compound of formula (I), which contributes to the production of the structure of the lipid-based nanocarrier composition.

Preferably, a zwitterionic co-lipid will be used.

Said co-lipid can be, for example, chosen from dioleylphosphatidylcholine (DOPC), dioleylphosphatidyluridinephosphatidylcholine (DOUPC) or dioleylphosphatidylethanolamine (DOPE).

These compounds can play the role of co-lipid when they are used in a mixture with a compound of formula (I). Alternatively, they can be included in formula (I), such as, for example, dioleylphosphatidyluridinephosphatidylcholine (DOUPC). In this case, they will either play the role of a compound of formula (I) or, in combination with another compound of formula (I), the role of co-lipid.

For the preparation of the compounds of formula (I), reference can be made to WO 2005/116043, which describes different access routes to this type of compounds (see in particular pp. 8-17 and the examples), as well as to WO2009/098404 or WO 2010/136676.

Preferably, the lipid-based nanocarrier composition according to the invention contains a plurality of metal nanoparticles, in particular 10 to 90 % (w/w) of metal nanoparticles, preferably 50 to 80%.

The metal nanoparticles contain, preferably, metal oxides which can be, for example, selected from iron oxide (magnetite Fe₃O₄, maghemite γ-Fe₂O₃, or other Co ferrite or Ni ferrite) which is known for its magnetic properties while metals (Au, Ag, Cu, Si, Ge, Fe, Co etc), metal alloys (FeCo, FePt, CoPt, FeBi etc.) and metal chalcogenides (CdS, CdSe, CdSe, ZnS...etc.) can be used for their plasmonic, luminescent or magnetic properties which are interesting in the aim to the development of a contrast agent or a labelling tool for bioimaging, tacking or sensoring.

Preferably, the metal nanoparticles have an overall size (average diameter) of approximately from 2 nm to 20 nm, preferably 5 to 10 nm.

The metal nanoparticles contained in the lipid-based nanocarrier compositions according to the invention may be mono-disperse (for example, they consist of a population having an average diameter centered around 10 nm), or mono- and/or poly-disperse (such as, for example, two populations having an average diameter centered around 4 nm and 10 nm), or else poly-disperse (different populations having different average diameters ranging from 2 nm and 20 nm).

Iron oxide nanoparticles are preferred.

The metal nanoparticles contained in the lipid-based nanocarrier compositions according to the invention can be, for instance, surface functionalized nanoparticles, or any type of nanoparticle presenting surface reactive groups, with a size comprised between 2 nm and 20 nm, preferably 5 to 10 nm, having a surface modified by grafting of amphiphilic or fatty ligand composed of an anchoring function - mainly carboxylates, phosphonate groups used for metal oxide and amine, thiol functions or other strong nucleophilic functions for metal nanoparticles such as gold nanoparticles - and a hydrophobic moiety which could be saturated or unsaturated hydrocarbon chains or perfluorinated carbon chains.

For example, coprecipited hydrophobic iron oxide nanoparticles have been prepared as previously published (G.A. van Ewijk, G.J. Vroege, A.P. Philipse, Convenient preparation methods for magnetic colloids, J. Magn. Magn. Mater. 201 (1999) 31-33) using stearic acid as fatty acid.

In a preferred embodiment the metal nanoparticles have a surface functionalization which provides them with hydrophobic properties. For instance they bear fatty acid residues on their surface.

By « therapeutic agent » is meant, for example, a natural or synthetic molecule used for preventing or treating a pathological condition, or restoring a biological function, in vitro or in vivo, in particular in animals, in particular in human beings, or else in isolated cells.

Advantageously, said therapeutic agent can be selected, for example, from anti-tumoral agents, antibiotic agents, anti-microbial agents, analgesic agents, anti-histaminic agents, bronchodilators agents, agents which are active on the central nervous system, anti-hypertension agents or agents which are active on the cardiovascular system (in particular anti-atherosclerosis agents), nucleic acids and their fragments; peptides, oligopeptides, proteins, antigens, antibodies or else stem cells etc.

Anti-cancer agents are of particular interest, such as, for example, sorafenib, sunitinib, taxanes (docetaxel, paclitaxel, cabazitaxel...), doxorubicine, adriamycin, daunomycin, melphalan, gemcitabine, cisplatin, derivatives of gemcitabine, or derivative of cisplatin, imatinib (Gleevec®), 5-fluorouracil, 9-aminocamptothecin, amine-modified geldanamycin, Taxol®, procarbazine or hydroxyurea.

Anti-atherosclerosis agents, such as, for example, tocopherols, resveratrol, anthocyanes, succinobucol, prostacycline, terutroban, picotamide, varespladib, darapladib, fumagilline, chemokins (CXCR3, IL10), aliskiren, lisinopril, losartan, irbesartan, telmisartan, epleronone, apolipoprotéine A1 (APOA-1) or glutathion are also of particular interest.

The invention also relates to a process for preparing a lipid-based nanocarrier composition comprising metal nanoparticles and at least one therapeutic agent, as described above, which comprises the following steps:
- preparing a solution containing at least one compound of formula (I), at least one metal nanoparticle and at least one therapeutic agent in an organic solvent
- adding this solution to an aqueous medium while stirring,
- sonicating the resulting aqueous solution and evaporating the organic solvent, and
- recovering the lipid-based nanocarrier composition thus obtained.

Preferred reaction conditions are as follows:
- the organic solvent can be selected, for example, from ether, choloroform, methylene chloride, ethyl acetate, butanol, propanol, ethanol and methanol;
- the aqueous medium is water;
- the evaporation of the organic solvent is carried out under reduced pressure, namely, for example, the pressure is reduced from 1 bar to 5 mbar;
- the lipid-based nanocarrier composition is recovered by centrifugation, for example at 14000 rpm during 15 to 30 min.

After centrifugation, the pellet containing the lipid-based nanocarrier composition may be re-suspended in water.

Preferably, when contained in the lipid-based nanocarrier compositions according to the invention as defined above, the therapeutic agent will be present at a concentration of about of 0.1 ng/mL to 10 mg/mL, and metal nanoparticles at a concentration of about of 0.1 ng/mL to 10 mg/mL, in the aqueous phase.

The invention also relates to the use of a lipid-based nanocarrier composition as an agent for transportation, vectorization, cellular delivery cellular targeting or cellular localization of at least one therapeutic agent, in particular for imaging guided therapy.

For example, the lipid-based nanocarrier composition according to the invention may be used in various therapeutic fields relating to diseases and/or disorders such as cancers, atherosclerosis, viral and non-viral infections, immunity disorders, inflammation etc.

The invention also concerns pharmaceutical compositions containing a lipid-based nanocarrier composition as described above and a pharmaceutically acceptable carrier.

The invention is illustrated non-limitatively by the examples below.

All commercially available reagents and solvents (Fluka, Sigma-Aldrich, Alfa-Aesar) were used without further purification.

Column chromatography was performed with flash silica gel (0.04-0.063 mm, Merck) or LH20 size-exclusion column (Sephadex LH-20)

The following abbreviations are used :
- 1,2-Dioleyl-sn-Glycero-3-phosphocholine (DOPC) (CAS Registry Number: 4235-95-4)
- N-[5'-(2',3'-dioleoyl)uridine]-N',N',N'-trimethylammonium (DOTAU) (CAS Registry Number: 868226-06-6)
- Thymidine 3'-(1,2-dipalmitoyl-*sn*-glycero-3-phosphate) (also called diC16dT), CAS Registry Number: 1160002-70-9 and
- Poly(oxy-1,2-ethanediyl), α-hydro-ω-methoxy-, ester with uridine 5'-(hydrogen butanedioate) 2',3'-di-(9Z)-9-octadecenoate (DOU-PEG) (CAS Registry Number: 1353570-75-8)

Figure 1 shows the size distribution by intensity measured by Dynamic Light Scattering (DLS) (figure 1A) and the zeta potential distribution (figure 1 B) of DOTAU nanoparticles (control).

Figure 2 shows the size distribution by intensity measured by Dynamic Light Scattering (DLS) (figure 2A) and the Zeta potential distribution (figure 2B) of nanoparticles of diC16dT (control).

Figure 3 shows the size distribution by intensity measured by Dynamic Light Scattering (DLS) (figure 3A) and the Zeta potential distribution (figure 3B) of a lipid-based (DOTAU) nanocarrier composition containing α-tocopherol.

Figure 4 shows the size distribution by intensity measured by Dynamic Light Scattering (DLS) (figure 4A) and the Zeta potential distribution (figure 4B) of a lipid-based (diC16dT, DOPC and DOU-PEG2000) nanocarrier composition containing prostacycline (PGI2.Na).

Figure 5 shows a SDS Page gel experiment of a lipid-based (DOTAU) nanocarrier composition containing Apolipoprotein-A1 (APOA1).

Figure 6 shows the size distribution by intensity measured by Dynamic Light Scattering (DLS) (figure 6A) and the Zeta potential distribution (figure 6B) of a lipid-based (DOTAU) nanocarrier composition containing Apolipoprotein-A1 (APOA1).

Figure 7 shows the particule size of iron oxide nanoparticles clusters encapsulated by DOTAU or diC16dT (preparations 1 and 2) or of a DOTAU-based nanocarrier composition comprising iron oxide nanoparticles and α-tocopherol (example 1) determined with a Zetasizer 3000 HAS MALVERN.

Figure 8 shows the detection standard curve for DOTAU (figure 8A) and α-tocopherol (figure 8B).

Figure 9 shows the HPLC analysis of iron oxide nanoparticles clusters encapsulated by DOTAU prepared in preparation 1 and of a DOTAU-based nanocarrier composition comprising iron oxide nanoparticles and α-tocopherol prepared in example 1.

### Preparation 1: Encapsulation of iron oxide nanoparticles clusters by DOTAU (control)

100 µL of stock solution of positively charged nucleolipid (DOTAU), (50 mg/mL in ether) and 20 µL of stock solution of iron oxide nanoparticles (10 mg/mL in ether) were mixed. DOTAU was prepared according to P. Chabaud et al., Bioconjugate Chem., 2006, 17, 466-472. The organic phase was added dropwise into the aqueous phase (2 mL of Milli-Q Water) placed in glass tube under stirring by vortex. Then the mixture was placed in glass flask.

Ether was removed under vacuum and the resulting crude material solution was sonicated for 3x15 min time and purified on LS columns to give pure solution of nanoparticles.

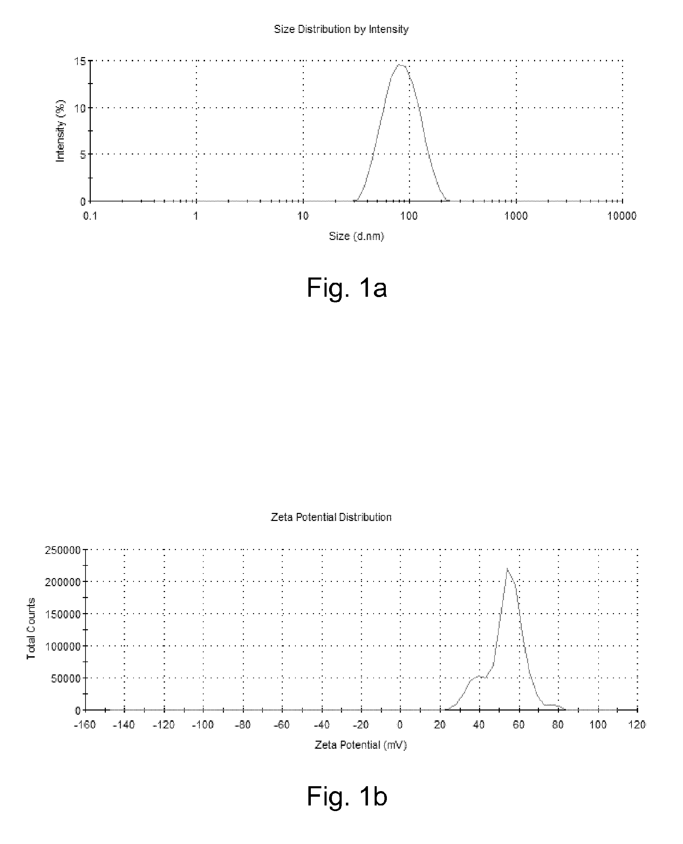

The size distribution by intensity measured by Dynamic Light Scattering (DLS) (d=80 nm) and the Zeta potential distribution measured with a MalvernNanoZS device (Zeta potential = +55 mV) are shown on figures 1A and 1B.

Preparation 2: Encapsulation of iron oxide nanoparticles clusters by diC16dT (control)

75 µL of stock solution of negatively charged nucleolipid (diC16dT) (50 mg/mL in chloroform), 25 µL of stock solution of 1,2-Dioleoyl-sn-Glycero-3-Phosphocholine (DOPC) (Avanti Polar lipids, 50 mg/mL in chloroform), 30 µL of stock solution of neutral nucleolipid (DOU-PEG2000) (10 mg/mL in chloroform) and 20 µL of stock solution of iron oxide nanoparticles (10 mg/mL in chloroform) were mixed. DIC16dT was prepared as disclosed in WO2010/13676. DOU-PEG2000 was prepared according to K.Oumzil et al., 2011, J. Control. Release, doi:10.1016/j.jconrel.2011.02.008.

The organic phase was added dropwise into the aqueous phase (2 ml of Milli-Q Water) placed in glass tube under stirring by vortex. Then the mixture was placed in glass flask.

Chloroform was removed under vacuum and the resulting crude material solution was sonicated for 3x15 min and purified on LS columns to give pure solution of nanoparticles.

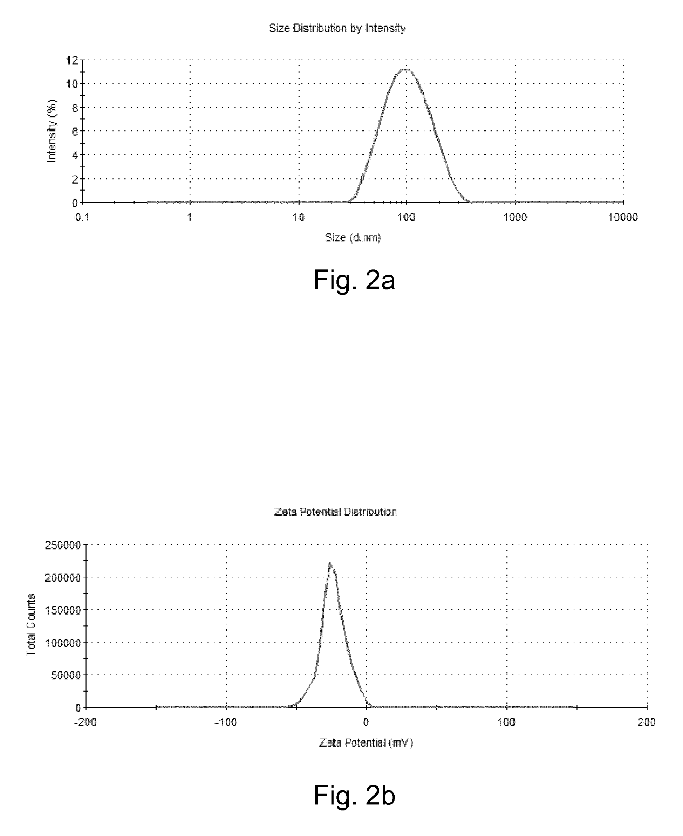

The size distribution by intensity measured by Dynamic Light Scattering (DLS) (d= 92 nm) and the Zeta potential distribution measured with a MalvernNanoZS device (Zeta potential = -23.6 mV) are shown on figures 2A and 2B.

### Example 1: Preparation of a lipid-based (DOTAU) nanocarrier composition containing iron oxide nanoparticles and α-tocopherol

100 µL of stock solution of positively charged nucleolipid (DOTAU) (50 mg/mL in ether), 10 µL of stock solution of α-tocopherol (Sigma Aldrich, 50 mg/mL in ether) and 20 µL of stock solution of iron oxide nanoparticles (10 mg/mL in ether) were mixed. The organic phase was added dropwise into the aqueous phase (2 mL of Milli-Q Water) placed in glass tube under stirring by vortex. Then the mixture was placed in glass flask.

Ether was removed under vacuum and the resulting crude material solution was sonicated for 3x15 min and purified on LS columns to give pure solution of nanoparticles.

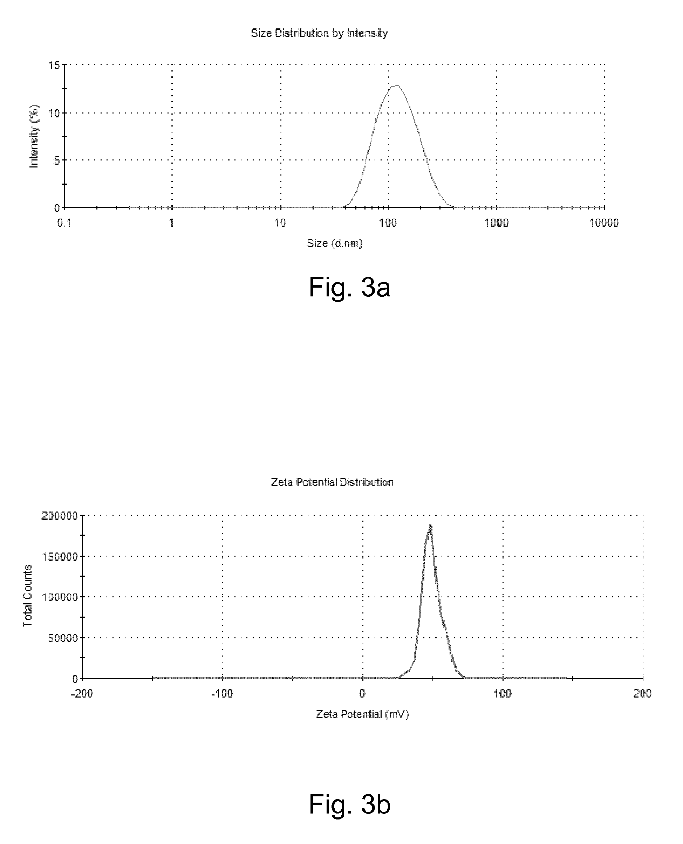

The size distribution by intensity measured by Dynamic Light Scattering (DLS) (d= 108 nm) and the Zeta potential distribution measured with a MalvernNanoZS device (Zeta potential = +49,2 mV) are shown on figures 3A and 3B.

### Example 2: Preparation of a lipid-based (diC16dT, DOPC and DOU-PEG2000) nanocarrier composition containing iron oxide nanoparticles and prostacycline (PGI2.Na)

75 µL of stock solution of negatively charged nucleolipid (diC16dT), (50 mg/mL in chloroform + 2% Et₃N), 25 µL of stock solution of DOPC (50 mg/mL in chloroform + 2% Et₃N), 30 µL of stock solution of neutral nucleolipid (DOU-PEG2000) (10 mg/mL in chloroform + 2% Et₃N), 1 mg of PGI2.Na (Sigma Aldrich) and 20 µL of stock solution of iron oxide nanoparticles (10 mg/mL in chloroform + 2% Et₃N) were mixed. The organic phase was added dropwise into the aqueous phase (2 ml of carbonate-bicarbonate buffer, pH 9.6 at 25 C) placed in glass tube under stirring by vortex. Then the mixture was placed in glass flask.

Chloroform was removed under vacuum and the resulting crude material solution was sonicated for 3x15 min and purified on LS column to give pure solution of nanoparticles.

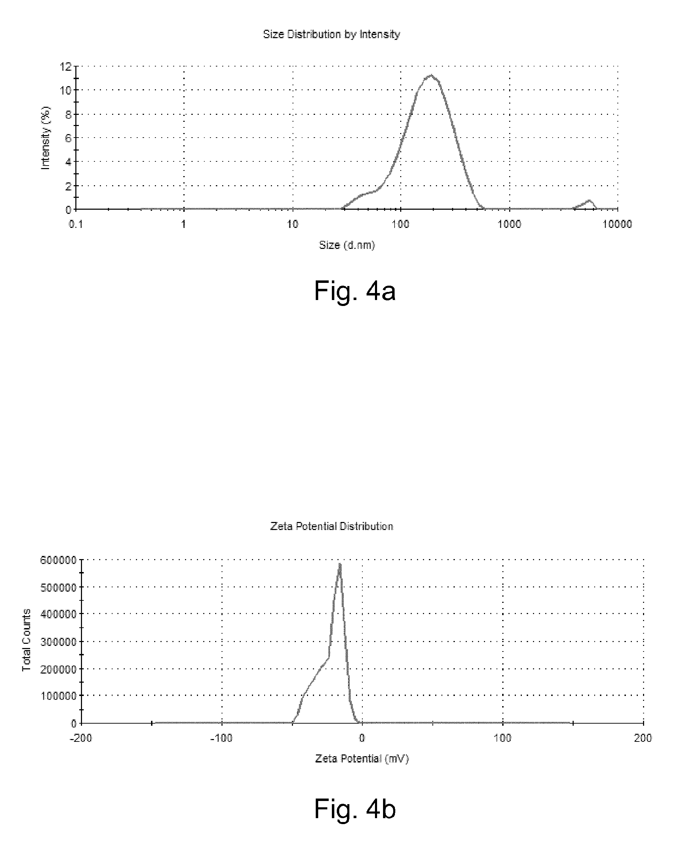

The size distribution by intensity measured by Dynamic Light Scattering (DLS) (d= 154 nm) and the Zeta potential distribution measured with a MalvernNanoZS device (Zeta potential = +-22.6 mV) are shown on figures 4A and 4B.

### Example 3: Preparation of a lipid-based (DOTAU) nanocarrier composition containing Apolipoprotein-A1 (APOA1)

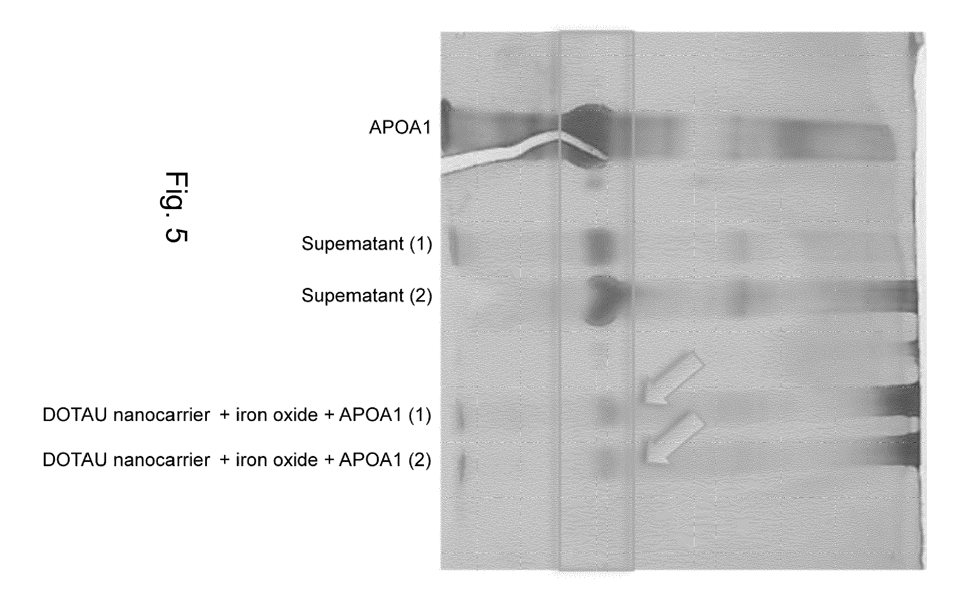

100 µL of stock solution of positively charged nucleolipid (DOTAU) (50 mg/mL in ether) and 20 µL of stock solution of iron oxide nanoparticles (10 mg/mL in ether) were mixed. The organic phase was added dropwise into the aqueous phase (100 µg of APOA1 (Sigma Aldrich) in 2 ml of Milli-Q Water) placed in glass tube under stirring by vortex. Then the mixture was placed in glass flask. Ether was removed under vacuum and the resulting crude material solution was sonicated for 3x15 min and purified on magnetic LS columns to give pure solution of nanoparticles. To demonstrate the presence of the APOA1 protein in the nanoparticles a SDS Page gel experiment was realised. The gel shows that the protein is present in the nanoparticles (two experiments, Figure 5).

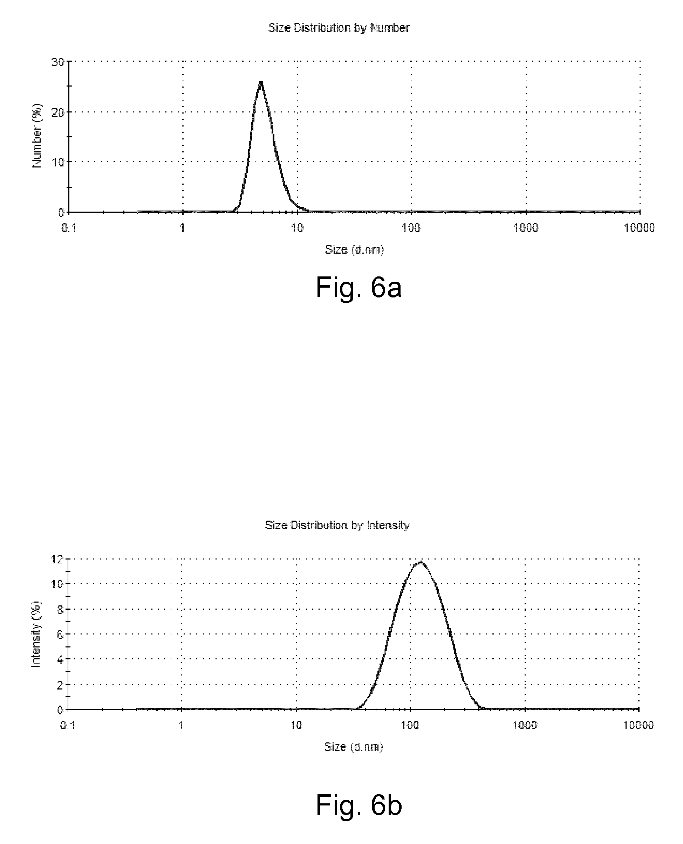

The size distribution by number measured by Dynamic Light Scattering (DLS) measured with a MalvernNanoZS device of APOA1 (d= 5.01 nm) and of the lipid-based (DOTAU) nanocarrier composition containing APOA1 (d= 105 nm) and the size distribution by number measured by intensity are shown on figures 6A and 6B.

### Example 4: Stability study

Iron oxide nanoparticles clusters encapsulated by DOTAU or diC16dT (preparations 1 and 2) and DOTAU-based nanocarrier composition comprising iron oxide nanoparticles and α-tocopherol (example 1) in 500 µL of Milli-Q water were incubated at 37°C under a 500rpm stirring. For different times (0, 1, 3, 6, 24, 48h), particle sizes were determined using a Zetasizer 3000 HAS MALVERN.

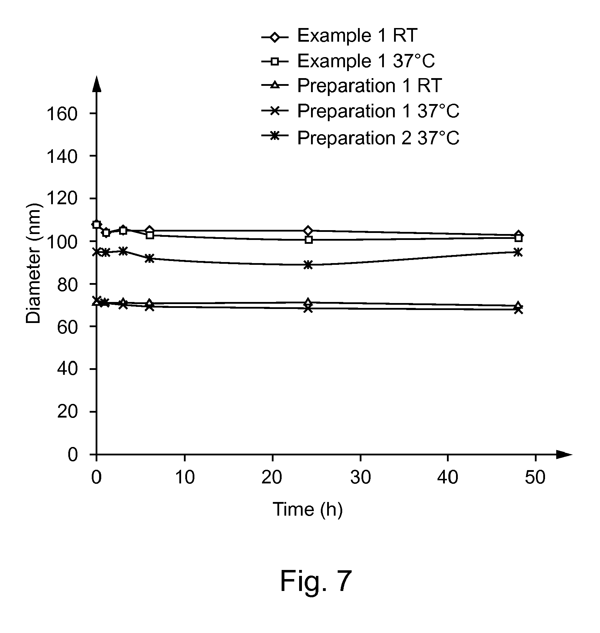

The results are shown on figure 7.

The results show that the overall sizes, either in absence or presence of therapeutic agents, and either positively or negatively charged, are not modified as a function of time (more than 2 days), which indicates colloidal stability both at room temperature and at 37°C.

### Example 5: Preparation of samples for HPLC analysis and dosage of DOTAU and α-tocopherol

Pure suspensions of cationic nanoparticles prepared in preparation 1 and example 1 were centrifuged at 14000 rpm for 15 min in order to remove the supernatant. Cationic nanoparticles (in the form of pellet) were suspended in ethanol. The resulting solution was mixed for 15 min at RT and centrifuged at 14000 rpm for 5 min. Then the supernatant (ethanol) was analyzed by HPLC.

A reverse phase U-HPLC method was developed for nucleolipid (DOTAU) and α-tocopherol quantification from the lipid-based nanocarrier composition containing iron oxide nanoparticles. This method allows the separation of the DOTAU and lipid-based (DOTAU) nanocarrier composition within 5 min.

The separation was carried out with a column Syncronis C18 50x2.1 mm, 1.7 µm with a mobile phase composed of MeOH + 0.1% HCOOH. The flow rate was set to 0.2 mL/min. The detection was performed at 293 nm and 260 nm for α-tocopherol and DOTAU, respectively. The injected volume was 1.0 µL, which allows the detection of DOTAU and α-tocopherol at thresholds of 5 ng and 15 ng, respectively.

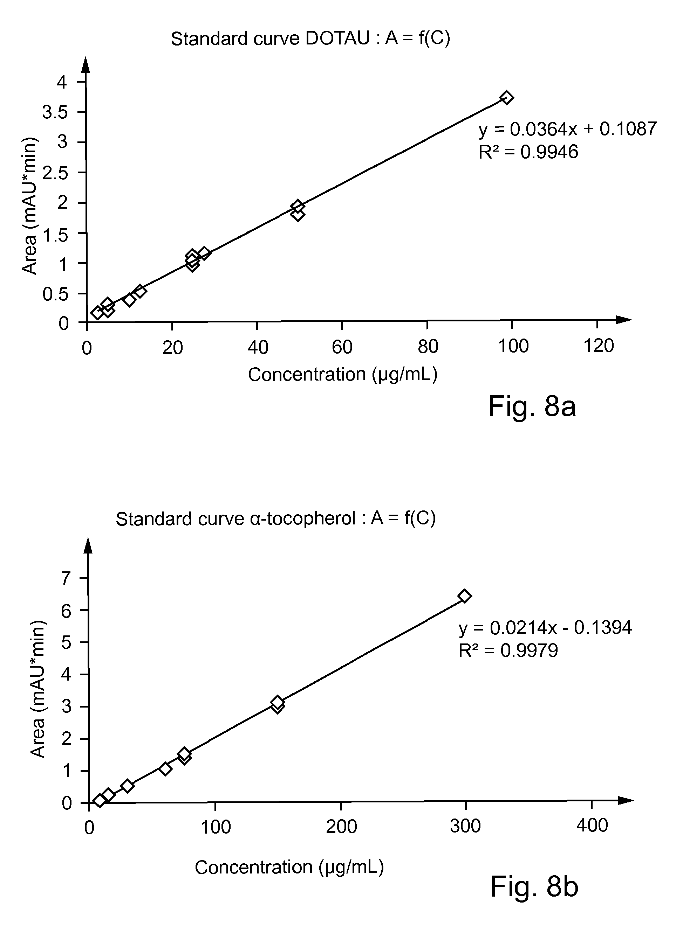

Standard curves for DOTAU and α-tocopherol, as shown on figures 8A and 8B, were generated by determining the intensity of signal versus concentrations.

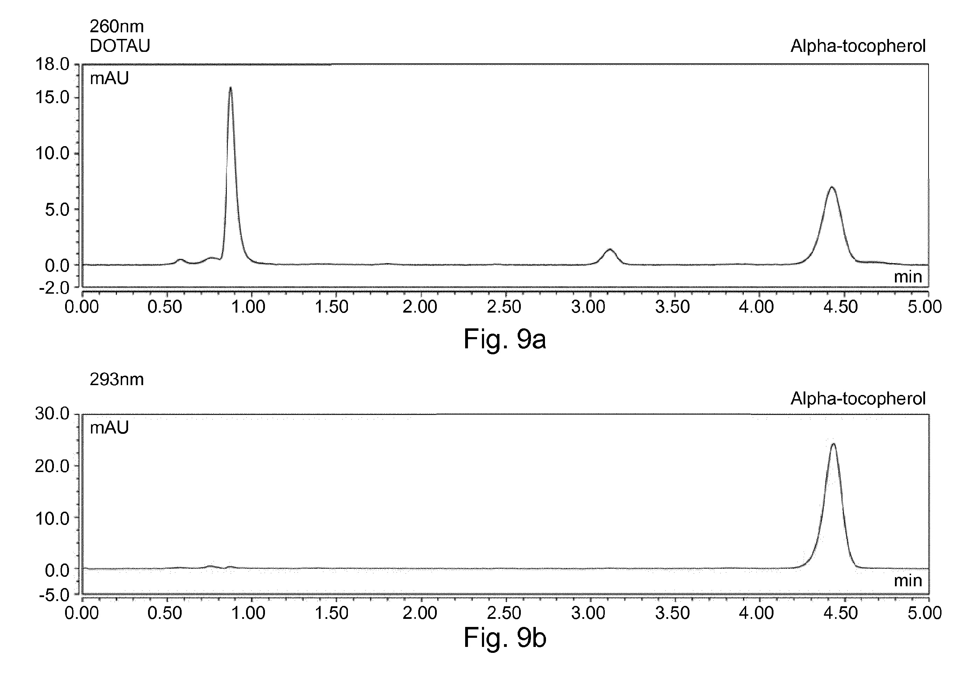

The HPLC analysis is shown on figures 9A (260 nm) and 9B (293 nm).

Quantification of both DOTAU and α-tocopherol was then possible, which led to encapsulated recovery and determination of loading ratio values. Loading ratio was 38% that obtained in the case of a DOTAU/ α-tocopherol (example 1) and the encapsulated drug recovery was around 10%., as shown in Table 1 below.

**Table 1**

| Sample No | Molecule | Mass in the supernatant (mg) | Mass in the pellet (mg) | Loading ratio | Recovery ratio |
|---|---|---|---|---|---|
| 1 | DOTAU 5mg | 2.80 | 0.12 | | |
| 2 | DOTAU 5mg | 4.02 | 0.12 | 30% | 10% |
| | alphatocopherol 0.5 mg | 0.52 | 0.05 | | |
| 3 | DOTAU 5mg | 3.61 | 0.11 | 38% | 6% |
| | alphatocopherol 1 mg | 0.93 | 0.06 | | |

This method has the advantage of using a low amount for analysis and a short analysis time, and has compatibility with mass spectrometry, which can be useful for biological analysis.

## Claims

1. A lipid-based nanocarrier composition comprising
a) at least one compound of formula (I) 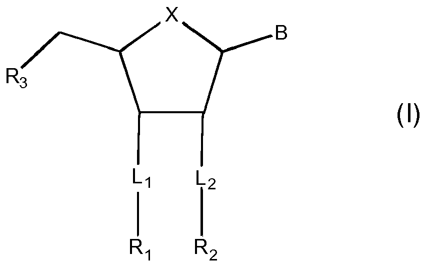 in which
- X is an oxygen atom, a sulfur atom or a methylene group,
- B is a purine or pyrimidine base, or else a non-natural mono- or bi-cyclic heterocyclic base, each ring of which comprises 4 to 7 members, optionally substituted ;
- L₁ and L₂, identical or different, represent hydrogen, an oxycarbonyl -O-C(O)-group, a thiocarbamate -O-C(S)-NH- group, a carbonate -O-C(O)-O- group, a carbamate -O-C(O)-NH- group, an oxygen atom, a phosphate group, a phosphonate group or a heteroaryl group comprising 1 to 4 nitrogen atoms, unsubstituted or substituted by a linear or branched, saturated or unsaturated C₂-C₃₀ hydrocarbon chain,
- or also, L₁ and L₂, together, form a ketal group of formula 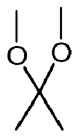
- or also L₁ or L₂ represents hydrogen, and the other represents a hydroxy group or a heteroaryl group comprising 1 to 4 nitrogen atoms, unsubstituted or substituted by a linear or branched C₂-C₃₀ alkyl chain,
- R₁ and R₂, identical or different, represent
- a linear or branched C₂-C₃₀ hydrocarbon chain, saturated or partially unsaturated, or
- aC₂-C₃₀ acyl chain,
- a diacyl chain in which each acyl chain is C₂-C₃₀,
- a diacylglycerol in which each acyl chain is C₂-C₃₀, or
- a sphingosine or ceramide group in which each acyl chain is C₂-C₃₀, or
- when L₁ or L₂ represents hydrogen, and the other represents a hydroxy group or a heteroaryl group comprising 1 to 4 nitrogen atoms, R₁ and R₂ do not exist;
- R₃ represents
- a hydroxy, amino, phosphate, phosphonate, phosphatidylcholine, O-alkyl phosphatidylcholine, thiophosphate, phosphonium, NH₂-R₄, NHR₄R₅ or NR₄R₅R₆ group in which R₄, R₅ and R₆, identical or different, represent a hydrogen atom or a linear or branched C₁-C₆ alkyl chain or linear or branched C₁-C₆ hydroxyalkyl, or
- a linear or branched C₂-C₃₀ alkyl chain, optionally substituted by a hydroxy group, or
- a heteroaryl group containing 1 to 4 nitrogen atoms, unsubstituted or substituted by a C₂-C₃₀ alkyl, optionally substituted by a hydroxy group; or by a (CH₂)ₘ-O-(CH₂)ₚ-R₉ group in which m = 1 to 6 and p = 0 to 20 and R₉ represents hydrogen or a cyclic ketal group containing 5 to 7 carbon atoms, unsubstituted or substituted by at least one linear or branched C₂-C₃₀ alkyl, or by a sterol radical,
or
- a -O-C(O)-(CH₂)_{q}-C(O)-O [(CH₂)₂-O]ᵣ-H group in which q is an integer from 2 to 6 and r is an integer from 4 to 30, preferably from 10 to 20, or also
- R₃ is bound by a covalent bond to another substituent R₃, identical or different, of another compound of formula (I), identical or different, in order to form a compound of formula (I) in the form of a dimer,
b) at least one metal nanoparticle, and
c) at least one therapeutic agent.

2. A lipid-based nanocarrier composition according to claim 1, comprising at least one compound of formula (I) in which at least one condition is fulfilled:
- X is an oxygen atom;
- B is thymine or uracile;
- L₁ and L₂ are oxycarbonyl -O-C(O)- groups which are substituted by a linear or branched C₂-C₃₀ hydrocarbon chain, saturated or partially unsaturated;
- L₁ is a phosphate group which is substituted by diacylglycerol in which each acyl group is C₂-C₃₀ and L₂ is hydrogen;
- R₃ is hydroxy, a NR₄R₅R₆ group in which R₄, R₅ and R₆ represent a hydrogen atom or a -O-C(O)-(CH₂)_{q}-C(O)-O [(CH₂)₂-O]ᵣ-H group in which q is 2 to 6 and r is an integer from 4 to 30, preferably from 10 to 20.

3. A lipid-based nanocarrier composition according to claim 1 or 2, wherein in formula (I), in the definitions of L₁, L₂, R₁ R₂ or R₃, the linear or branched alkyl chain is C₈-C₂₆, preferably C₁₆-C₂₀; and/or the linear or branched C₂-C₃₀ hydrocarbon chain is C₈-C₂₆, preferably C₁₆-C₂₀; and/or the C₂-C₃₀ acyl chain is C₈-C₂₆, preferably C₁₆-C₂₀.

4. A lipid-based nanocarrier composition according to any one of claims 1 to 3, comprising at least one compound of formula (I), selected from
- N-[5'-(2',3'-dioleoyl)uridine]-N',N',N'-trimethylammonium
- Thymidine 3'-(1,2-dipalmitoyl-*sn*-glycero-3-phosphate), and
- Poly(oxy-1,2-ethanediyl), α-hydro-ω-methoxy-, ester with uridine 5'-(hydrogen butanedioate) 2',3'-di-(9Z)-9-octadecenoate.

5. A lipid-based nanocarrier composition according to any one of claims 1 to 4, which comprises at least 2 different compounds of formula (I).

6. A lipid-based nanocarrier composition according to any one of claims 1 to 5, which comprises a plurality of metal nanoparticles.

7. A lipid-based nanocarrier composition according to any one of claims 1 to 6, in which the metal nanoparticle contains metal oxides, metals, metal alloys or metal chalcogenides.

8. A lipid-based nanocarrier composition according to any one of claims 1 to 7, in which the metal nanoparticle contains iron oxide.

9. A lipid-based nanocarrier composition according to any one of claims 1 to 8, in which the metal nanoparticles have an overall size of 2nm to 20nm.

10. A lipid-based nanocarrier composition according to any one of claims 1 to 9, in which the population(s) of metal nanoparticles is/are either monodisperse, or monodisperse and/or polydisperse, or else polydisperse.

11. A lipid-based nanocarrier composition according to any one of claims 1 to 10, in which the therapeutic agent is selected from anti-tumoral agents, antibiotic agents, anti-microbial agents, analgesic agents, anti-histaminic agents, bronchodilators agents, agents which are active on the central nervous system, anti-hypertension agents or agents which are active on the cardiovascular system, anti-atherosclerosis agents, nucleic acids and their fragments; peptides, oligopeptides, proteins, antigens, antibodies or stem cells.

12. A lipid-based nanocarrier composition according to any one of claims 1 to 11, in which the therapeutic agent is selected from anti-tumoral agents and anti-atherosclerosis agents.

13. A process for preparing a lipid-based nanocarrier composition comprising metal nanoparticles according to any one of claims 1 to 12, which comprises the following steps:
- preparing a solution containing at least one compound of formula (I), at least one metal nanoparticle and at least one therapeutic agent in an organic solvent
- adding this solution to an aqueous medium while stirring,
- sonicating the resulting aqueous solution and evaporating the organic solvent, and
- recovering the lipid-based nanocarrier composition thus obtained.

14. The use of a lipid-based nanocarrier composition according to any one of claims 1 to 12 as an agent for transportation, vectorization, cellular delivery, cellular targeting or cellular localization of at least one therapeutic agent.

15. Pharmaceutical compositions containing a lipid-based nanocarrier composition according to any one of claims 1 to 12 and a pharmaceutically acceptable carrier.
